# EUROPEAN PATENT APPLICATION

(11) **EP 1 731 191 A1**
(43) Date of publication of application: **13.12.2006**
(21) Application number: 05720047.9
(22) Date of filing: 04.03.2005
(51) Int. Cl.: A61M 25/02, A61L 31/00

(54) **CUFF MEMBER**

(30) Priority: 08.03.2004 JP 2004064562; 08.03.2004 JP 2004064563
(71) Applicant: Japan as represented by president of National Cardiovascular Center, Suita-shi, Osaka 565-8565 (JP); Bridgestone Corporation, Tokyo 104-8340 (JP)
(72) Inventor: TATSUMI, Eisuke, Suita-shi, Osaka5650851 (JP); MIZUNO, Toshihide, Takatsuki-shi, Osaka 5691046 (JP); TAENAKA, Yoshiyuki, Toyonaka-shi,Osaka 5600861 (JP); NEMOTO, Yasushi, Yokohama Plant, Bridgestone Corp., Yokohama-shi, Kanagawa 2440812 (JP); OKAMOTO, Y., Yokohama Plant, Bridgestone Corp., Yokohama-shi, Kanagawa 244081 (JP); TAZAWA, Hare, Technical Center, Bridgestone Corp., Kodaira-shi, Tokyo1870031 (JP)
(74) Representative: HOFFMANN EITLE
(86) International application number: PCT/JP2005/003775
(87) International publication number: WO 2005/084742

(57) **Abstract**

A cuff member that enhances the invasion and engraftment of cells from subcutaneous tissue of a living body and robustly adheres to the subcutaneous tissue through the vascularization of capillary vessels, and can consequently isolate a wound from the outside, block exacerbating factors such as bacterial infection during healing, inhibit the downgrowth, and reduce infection problems including tunnel infection. A cuff member 2 includes a flange 3 and a tubular portion 4. The cuff member 2 includes a three-dimensional network open-cell porous structure, which is formed of a thermoplastic resin or a thermosetting resin and has an average pore size of 100 to 1000 µm and an apparent density of 0.01 to 0.5 g/cm³. A pad 5 overlies the flange 3. A tube 6 passes through the pad 5 and the flange 3.

## Description

### Field of the Invention

The present invention relates to a cuff member that can be invaded by cells generated from body tissue and can robustly adhere to body tissue, and more particularly to a cuff member useful at an insertion point in a biological skin in therapy in which a cannula or a catheter is subcutaneously inserted, such as blood circulation using a ventricular assist device, peritoneal dialysis, intravenous hyperalimentation, gastrogavage, cannula DDS, and catheter DDS.

### Background of the Invention

Unlike a urethral catheter, gastrointestinal hyperalimentation, and airway management, in which a cannula or a catheter is placed in a vessel opened outside, in recently developed therapy, such as a ventricular assist device or a peritoneal dialysis, a subcutaneous tissue must be incised and a cannula or a catheter must be placed in a living body. If the cannula or the catheter is placed in the living body for a long period of time, a cuff member (also called skin cuff) is utilized to artificially close the insertion point to isolate the inside of the living body from the outside and thereby prevent a bacterium from invading the living body or prevent body fluid or water from volatilizing. Until now, in blood circulation using a ventricular assist device, an insertion cannula is wrapped with fabric velour typically formed of polyester fiber and is placed in the living body by suturing the fabric velour and subcutaneous tissue at the insertion point. Also in peritoneal dialysis, a cuff member, such as fabric velour formed of polyester fiber, is fixed at the insertion point of a catheter. The catheter is placed under the skin by suturing the subcutaneous tissue so that the cuff member is compressed. Certain fabric velour is impregnated with collagen or the like to achieve robust adhesion. Furthermore, a cuff member formed of a biocompatible material may be fixed in the subcutaneous tissue at the insertion point.

However, since the blood circulation using a ventricular assist device enhances the blood circulation with an extracorporeal pulsatile pump, the vibration of the pulsatile pump corresponding to about 1.5 Hz is transmitted to a cannula. Thus, the insertion point of the cannula is continuously under the mechanical load generated by the vibration. In addition, changes in patient's posture or the movement of a cannula during disinfection of the insertion point causes a peel stress at a bonding interface between the subcutaneous tissue and a cuff member. A typical trouble for which reduced adhesion between the cuff member and the subcutaneous tissue caused by the stress loading is concluded to be responsible is an infection problem such as tunnel infection. In cases of ventricular assist device therapy, such an infection problem is experienced more frequently. Considering the effects of the bacterial infection on a complication and cardiac failure, a cuff member for preventing the bacterial infection is urgently required in the ventricular assist device therapy.

Peritoneal dialysis, in which a catheter is inserted under the skin and is placed for a long period of time, also has a momentous issue concerning the cuff member. That is, in this therapy, a catheter is placed in an abdominal cavity to inject or discharge dialysate. However, a living body may recognize the catheter as foreign matter and therefore intends to reject the catheter. Thus, subcutaneous tissue and the catheter insufficiently adhere to each other. This causes a downgrowth phenomenon, in which epidermis enters the abdominal cavity along the catheter. This downgrowth pocket makes the access of an antiseptic solution difficult, is responsible for epidermal inflammation and tunnel infection, and eventually induces peritonitis. By consideration of a report showing that patients suffering from frequent pseudomonas peritonitis exhibit increased incidence of sclerosing encapsulating peritonitis (SEP), the improvement of the cuff member to prevent infection is a momentous issue in the peritoneal dialysis.

As described above, a collagen-based cuff member has been developed. However, such a cuff member absorbs a liquid, such as physiological saline, alcohol, Isodine, blood, and/or body fluid, to decrease in volume. Thus, it is difficult to grow the subcutaneous tissue at the catheter insertion point. Consequently, the cuff member cannot inhibit the downgrowth.

PCT Japanese Translation Patent Publication No. 4-502722 discloses a biocompatible resinous porous material that serves as a cuff material utilizing a sponge-like material in which subcutaneous tissue can grow. The porous material is disposed on an insert tube such as a catheter. However, in such a cuff material, while the porous material may be infiltrated with subcutaneous tissue and be organized, epidermis cannot be bonded to the porous material and grows under the subcutaneous tissue along the insert tube. Consequently, the downgrowth cannot be inhibited.

A cuff material in which a generally discoidal porous material and a tubular porous material are combined to infiltrate the disc surface with subcutaneous tissue has been devised. The discoidal porous material can be infiltrated with subcutaneous tissue and effectively inhibit the downgrowth. However, epidermis cannot be bonded to the discoidal porous material and grows along a catheter outside the body. Thus, a gap is formed between the epidermis and the discoidal porous material. Pus or a residue of an antiseptic solution may be accumulated in the gap and induce pocket infection.

In addition to the downgrowth, the ventricular assist device therapy has another momentous issue. Since subcutaneous tissue is insufficiently isolated from the outside at a cannula insertion point, the insertion point must not be dipped into water. Thus, as daily treatment to keep patient's body clean, a body surface is only wiped with sterilized cotton. Not only bathing but also shower are prohibited. This considerably decreases patient's quality of life (QOL).

### Summary of the Invention

The present invention was achieved in view of such problems of the related art. Accordingly, it is an object of the present invention to provide a cuff member unit that can prevent exposure due to the downgrowth and a cuff member for use in the cuff member unit. In particular, it is an object of the present invention to provide a cuff member that enhances the invasion and engraftment of cells from subcutaneous tissue of a living body and robustly adheres to the subcutaneous tissue through the vascularization of capillary vessels, and can consequently inhibit the downgrowth and reduce infection problems including tunnel infection. This cuff member can improve the isolation of subcutaneous tissue from the outside in the ventricular assist device therapy. Thus, a patient can take a shower.

A cuff member according to a first aspect includes a flange overlying the outer surface of a living body and a tubular portion standing on one side of the flange. The cuff member includes a three-dimensional network open-cell porous structure, which is formed of a base resin composed of a thermoplastic resin or a thermosetting resin and has an average pore size of 50 to 1000 µm and an apparent density of 0.01 to 0.5 g/cm³.

A cuff member according to a second aspect is a cuff member for a biological insert tube. The cuff member includes a first flange overlying a living body, a tubular portion standing on one side of the first flange, a second flange one side of which overlies the other side of the first flange, and a polymeric material pad overlying the other side of the second flange. The first flange and the tubular portion include a three-dimensional network open-cell porous structure formed of a base resin composed of a thermoplastic resin or a thermosetting resin. The three-dimensional network open-cell porous structure has an average pore size of 100 to 1000 µm and an apparent density of 0.01 to 0.5 g/cm³. The second flange includes a three-dimensional network open-cell porous structure formed of a base resin composed of a thermoplastic resin or a thermosetting resin. This three-dimensional network open-cell porous structure has an average pore size of 1 to 100 µm and an apparent density of 0.05 to 1 g/cm³.

Since the cuff members according to the first aspect and the second aspect have a three-dimensional network open-cell porous structure having the specific average pore size and the specific apparent density and formed of a thermoplastic resin or a thermosetting resin, the cuff members enhance the invasion and engraftment of cells into pores of the three-dimensional network open-cell porous structure and thereby can robustly adhere to body tissue.

The cuff members according to the first aspect and the second aspect enhance the invasion and engraftment of cells from subcutaneous tissue of a living body. Thus, the cuff members can robustly adhere to the subcutaneous tissue through the vascularization of capillary vessels. Consequently, the cuff members isolate a wound from the outside, block exacerbating factors such as bacterial infection during healing, inhibit the downgrowth, and reduce infection problems including tunnel infection.

The cuff members according to the first aspect and the second aspect can suitably be used at an insertion point in a biological skin in therapy in which a cannula or a catheter is subcutaneously inserted, such as blood circulation using a ventricular assist device, peritoneal dialysis, intravenous hyperalimentation, gastrogavage, cannula DDS, and catheter DDS.

In a cuff member using the cuff member according to the first aspect, a flange is covered with a pad, and the pad extends beyond the flange. Thus, it takes time for the downgrowth of skin to affect the tubular portion. In addition, such a structure can prevent water from entering subcutaneous tissue along a cannula.

In the cuff member according to the second aspect, the first flange covered with the polymer resin pad retards the downgrowth from affecting the tubular portion. In addition, such a structure can prevent liquid from entering subcutaneous tissue along a cannula. The first flange is placed under skin and is organized through invasion of subcutaneous tissue. The end of epidermis overlying the first flange is bonded to the edge of the second flange. Thus, implantation can be performed without the epidermis growing over the polymer resin pad. Epidermis is inhibited from growing under the polymer resin pad for a long period of time.

Consequently, the cuff member unit can be implanted in a living body for a long period of time without the effects of the downgrowth. A patient who receives the ventricular assist device therapy can take a shower without wetting an insertion cannula.

Furthermore, since the pad overlies the outer surface of a living body, vibration of a tube, such as pulsation, is transmitted to a living body via the pad. Thus, a stress applied to the living body through the tube is dispersed in a wider area. According to the second aspect, since epidermis is securely bonded to the second flange, implantation can be performed without drawing up epidermis over the pad. Furthermore, the epidermis is inhibited from growing under the pad. Thus, an infection pocket cannot be formed.

### Brief Description of the Drawings

Fig. 1 is a structure view of a cuff member according to an embodiment.
Fig. 2 is a cross-sectional view illustrating an application of the cuff member illustrated in Fig. 1.
Fig. 3 is an illustrative view of a comparative example.
Fig. 4 is a structure view of a cuff member according to another embodiment.
Figs. 5a and 5b are structure views of cuff members according to still another embodiments.
Fig. 6 is a typical SEM photograph of a cross section of a porous structure.

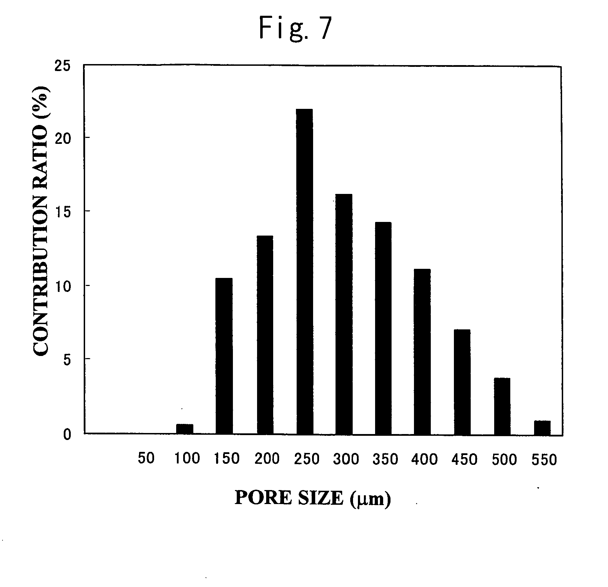

Fig. 7 illustrates the pore size distribution of a porous structure.
Fig. 8 is a photograph illustrating that epidermis of an adult goat and a pad are satisfactorily bonded to each other on a flange organized using the cuff member according to a first aspect.
Fig. 9 is a photograph illustrating infection and a downgrowth phenomenon along a tube 6 observed in a comparative example.
Figs. 10a and 10b are structure views of a cuff member unit according to an embodiment of a second aspect.
Fig. 11 is a cross-sectional view illustrating an application of the cuff member unit illustrated in Fig. 10.
Fig. 12 is a structure view of a cuff member unit according to another embodiment.
Figs. 13a and 13b are structure views of cuff member units according to still another embodiments.
Fig. 14 illustrates the pore size distribution of a porous structure of a second flange.
Fig. 15 is a photograph illustrating a cuff member embedded in an adult goat.
Fig. 16 is a photograph illustrating that epidermis of an adult goat and a pad satisfactorily are bonded to each other on a flange organized using the cuff member unit according to the second aspect.
Fig. 17 is a photograph illustrating a tissue invading a porous structure layer.
Fig. 18 is a photograph illustrating the cuff member according to the second aspect after three postoperative months.
Fig. 19 is a photograph illustrating the cuff member according to the second aspect after 12 postoperative months.
Fig. 20 is a photograph illustrating tissue specimens around the boundary between epidermis and the second flange of the cuff member according to the second aspect after 12 postoperative months.

### Detailed Description

A cuff member according to an embodiment of the present invention will be described in detail below.

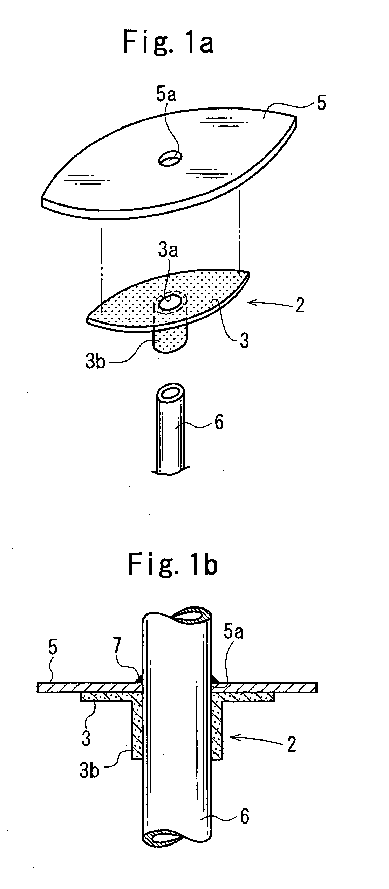

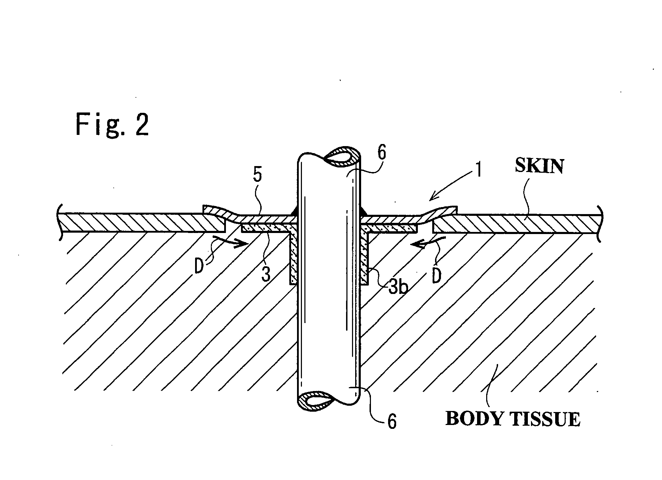

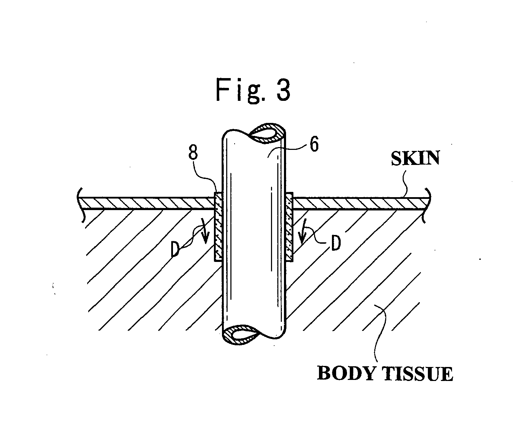

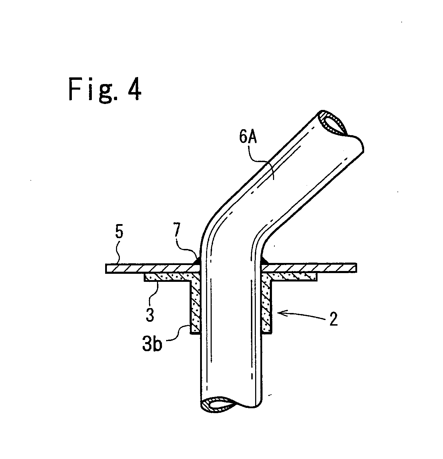

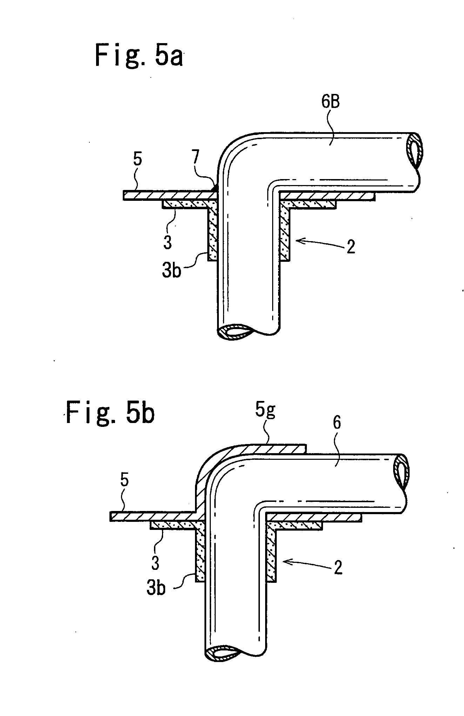

Fig. 1a is an exploded perspective view of a cuff member according to an embodiment of a first aspect. Fig. 1b is a longitudinal sectional view of this cuff member. Fig. 2 is a cross-sectional view illustrating an application of the cuff member. Fig. 3 is a cross-sectional view of a comparative example. Figs. 4 and 5 are longitudinal sectional views of cuff members according to other embodiments of the first aspect.

As illustrated in Fig. 1, a cuff member 2 includes a flange 3 and a tubular portion 3b standing on one side of the flange 3. The flange 3 has at its center a circular opening 3a having a diameter of about 5 to 100 mm and being coaxial with the tubular portion 3b. The cuff member may hold a plurality of tubes 6. For example, in a ventricular assist device therapy, two tubes 6 (cannulas) consisting of a transfusion tube and a blood drawing tube are placed in a patient. When a pad 5 has two openings 5a and the flange 3 has two openings 3a and two tubular portions 3b, one cuff member can hold two insert tubes. This may reduce aggression against a patient. Whether a transfusion tube and a blood drawing tube are inserted independently through two cuff members or cuff member units, or simultaneously through one cuff member or cuff member unit may appropriately be determined by a person skilled in the art in consideration of clinical significance, the condition of a patient, and invasiveness. Alternatively, a transfusion tube and a blood drawing tube may be inserted into a tube having a larger diameter than the transfusion tube and the blood drawing tube. The thicker tube, which is a so-called double-lumen tube, may be inserted through a cuff member or cuff member unit into a living body. As a matter of course, in therapies other than the ventricular assist device therapy, a plurality of linear structures, such as a power cord, a control cable, and a measuring cable for an artificial heart pump and a thin tube for DDS may be held in one tube and be inserted through a cuff member or cuff member unit.

The opening 3a has the same diameter as the inner size (diameter) of the tubular portion 3b.

The cuff member 2 is formed of a porous resin material that exhibits excellent adhesion to body tissue as described below. In the cuff member 2, the tubular portion 3b and the flange 3 are combined into one piece.

The flange 3 may have a planar shape of a circle, an ellipse, a lens, or a teardrop. In general, when skin is incised in a straight line with a scalpel, elliptical body tissue appears as illustrated in Fig. 1. Thus, the flange 3 is preferably elliptical to cover the exposed tissue efficiently. The thickness of the flange 3 depends on the physical strength of the flange 3 as well as complicated factors, such as the average pore size and the inclination of the cuff member 2 (these affect the depth of tissue invasion and the degree of differentiation) as described below. In general, the flange 3 suitably has a thickness of about 0.05 to 20 mm. When the flange 3 is circular, the flange 3 suitably has a diameter of about 10 to 200 mm. When the flange 3 is elliptical, lens-shaped, or teardrop-shaped, the major axis is preferably 10 to 200 mm and the minor axis is preferably about 5 to 80% of the major axis.

The tubular portion 3b suitably has a length of about 10 to 500 mm. The thickness of the tubular portion 3b depends on the physical strength of the tubular portion 3b as well as complicated factors, such as the average pore size and the inclination of the cuff member 2 (these affect the depth of tissue invasion and the degree of differentiation) as described below. In general, the tubular portion 3b suitably has a thickness of about 0.05 to 20 mm. The tubular portion 3b is not necessarily straight and may be bent freely from an insertion site along a tube.

The pad 5 overlies one side of the flange 3 of the cuff member 2. The pad 5 and the flange 3 are combined into one piece by bonding or the like. The pad 5 has a figure similar to the flange 3 and is preferably smaller than the flange 3. The pad 5 has an opening 5a coaxial with the opening 3a of the flange 3 and having the same size as the opening 3a.

The pad 5 is formed of one or at least two polymeric materials selected from the group consisting of a polyvinyl chloride resin, a polyurethane resin, a polyamide resin, a polylactic acid resin, a polyolefin resin, a polyester resin, a fluorocarbon resin, a urea resin, a phenol resin, an epoxy resin, a polyimide resin, a silicon resin, an acrylic resin, a methacrylate resin, chitin, chitosan, keratin, hyaluronic acid, fibroin, and their derivatives.

The thickness of the pad 5 may depend on the flexibility of the polymeric material and is suitably about 0.1 to 100 mm. A person skilled in the art may properly determine the thickness depending on the insertion site. For example, a relatively soft pad is used at the side of a body to follow the curved surface, and a relatively hard pad is used at the center of chest on the rib where the body surface is almost flat. The cuff member 2 is suitably used for applications in which the tube 6 is inserted from the outer surface of the body into the inside of the body.

The tube 6 is inserted through the openings 5a and 3a and the tubular portion 3b. The tube 6 may be bonded in a watertight manner to the pad 5 by fusion using a highfrequency wave, heat, laser, and an ultrasonic wave or with an adhesive. The pad 5 and part of or the entire tube 6 may be integrally molded by injection molding. In this embodiment, the pad 5 and the tube 6 securely adhere to each other with an adhesive 7.

As illustrated in Fig. 2, when the tube 6 is inserted into a living body using the cuff member 2, skin is incised to expose tissue. A small portion of subcutaneous tissue at the outer edge of the exposed tissue is peeled off to form a pocket. The body tissue is incised to insert the tube 6 into the body tissue. The flange 3 is laid on the outer surface of the body tissue. Together with the tube 6, the tubular portion 3b is embedded in the body tissue. The incised body tissue around the tube 6 is sutured when necessary. The first flange 3 overlies the exposed body tissue and is embedded in the pocket formed by peeling off the subcutaneous tissue, that is, is placed under the epidermis. A second flange 4 is laid on the edge of skin around the exposed body tissue. The cuff member 2 may be fixed on the body surface of a patient by sewing the second flange 4 and epidermis together. Because the second flange is formed of a soft three-dimensional network structure material, it can easily be sutured with an ordinary suture needle. Furthermore, an adhesive tape that is air permeable and is imperviousness to water (not shown) may be attached over the outer edge of the second flange 4 and skin around this outer edge. The adhesive tape can prevent water or other liquids from entering the body tissue.

As described above, when the tube 6 is inserted into the body tissue using the cuff member 2, the downgrowth of skin initially tends to proceed to the interface between the second flange 4 and the first flange. However, because the interface has no physical space for epidermis to enter, the downgrowth of skin tends to proceed in the direction of the outer edge of the first flange and eventually under the first flange. In other words, the epidermis tends to grow apart from the sutured portion. However, in the cuff member 2 according to the present invention, tissue that invaded the first flange from a body tissue side passes through the first flange and reaches the back of the epidermis. Thus, the epidermis linked to the body tissue is significantly inhibited from growing apart from the sutured portion on the first flange, and the epidermal end is maintained in close connection with the second flange.

Furthermore, stress transmitted from the tube 6 to a living body by pulsation of the tube 6 or the like is also transmitted to the living body via the pad 5. Thus, the stress is dispersed in a wider area. Hence, a stimulus applied to body tissue around the tube 6 is alleviated.

Fig. 3 illustrates a comparative example in which a cylindrical cuff member 8 formed of a material of the same type as the cuff member 2 is attached to the outer surface of the tube 6 and inserted into body tissue, as disclosed in PCT Japanese Translation Patent Publication No. 4-502722.

In this case, the downgrowth of skin proceeds into the body tissue along the cuff member 8, as indicated by arrow D. Thus, a large part of the cuff member 8 is exposed at the surface of the skin at an early stage. Furthermore, vibration of the tube 6, such as pulsation, is focused on an small area around the tube 6. A cuff member unit 1 illustrated in Fig. 1 or 2 can remove such a drawback.

While the tube 6 extends perpendicular to the outer surface of a body in Figs. 1a, 1b, and 2, it may obliquely extend as in a tube 6A illustrated in Fig. 4 or extend along the pad 5 as in a tube 6B illustrated in Figs. 5a and 5b. This may be properly determined by a person skilled in the art by consideration of the relationship between patient's posture and medical equipment to which an insert tube is connected and the weight and the width of movement of the tube 6, 6A, or 6B.

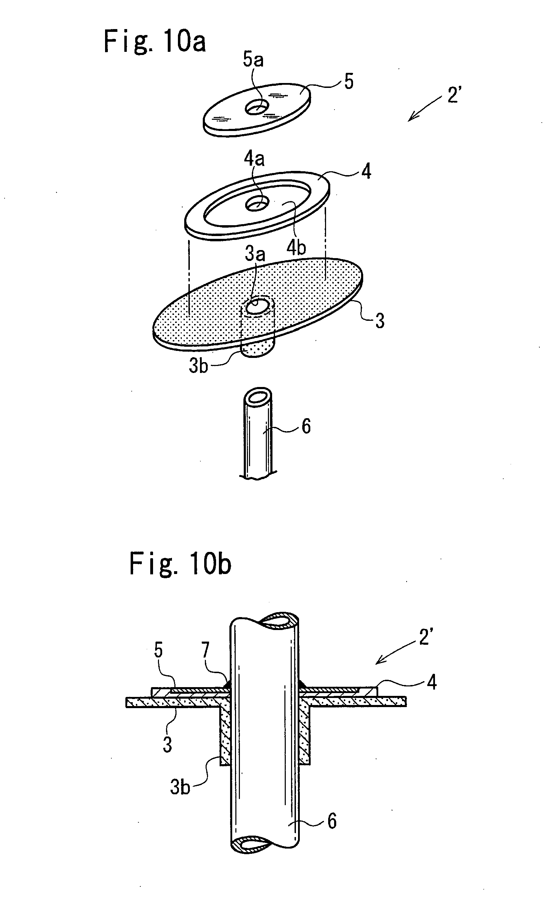

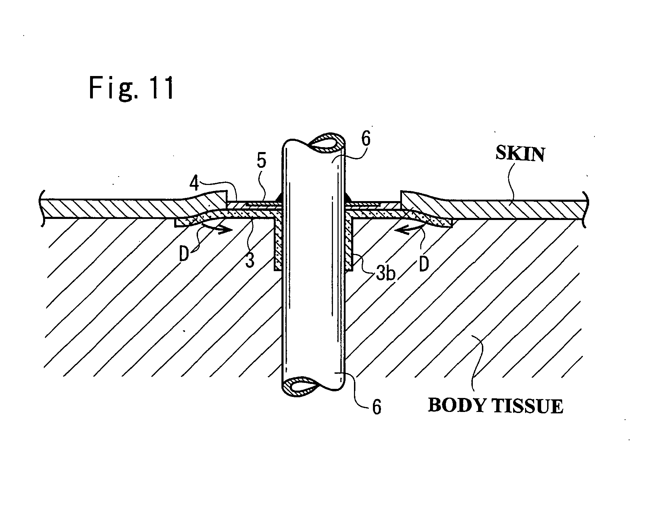

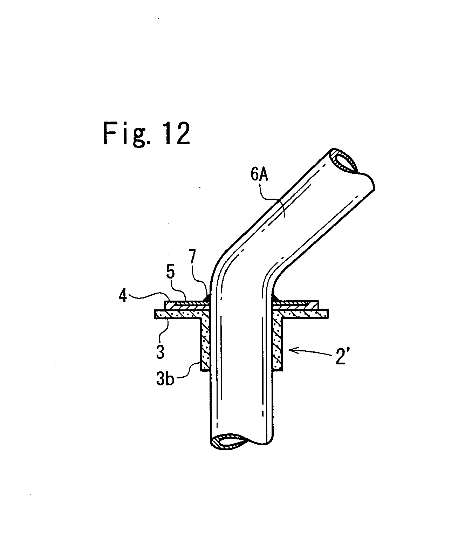

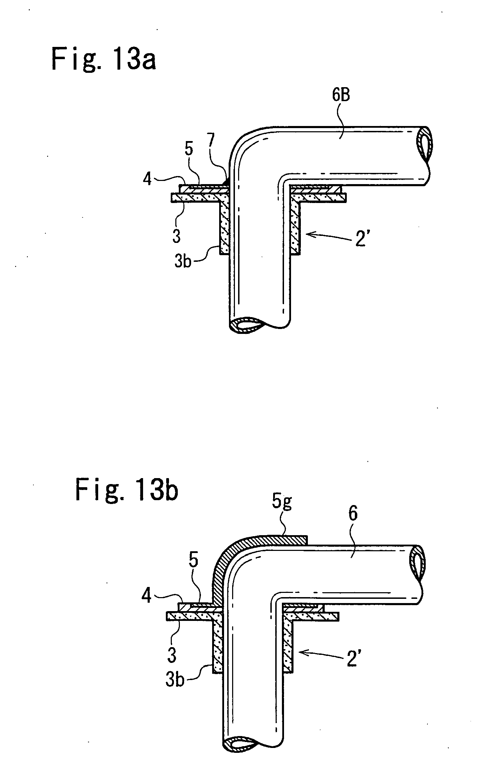

Fig. 10a is an exploded perspective view of a cuff member 2' according to an embodiment of a second aspect. Fig. 10b is a longitudinal sectional view of this cuff member. Fig. 11 is a cross-sectional view illustrating an application of the cuff member. Figs. 12 and 13 are longitudinal sectional views of cuff members according to other embodiments of the second aspect.

As illustrated in Figs. 10a and 10b, the cuff member 2' includes a first flange 3, a tubular portion 3b standing on one side of the first flange 3, a second flange 4, and a pad 5. The first flange 3 has at its center a circular opening 3a having a diameter of about 5 to 100 mm and being coaxial with the tubular portion 3b. The second flange 4 and the pad 5 have openings 4a and 5a coaxial with the opening 3a. The openings 4a and 5a have the same diameter as the opening 3a. Thus, the openings 3a, 4a, and 5a have the same diameter as the inner size (diameter) of the tubular portion 3b.

A tube 6 is inserted through the openings 3a to 5a and the tubular portion 3b. While the cuff member illustrated holds one tube 6, the cuff member may hold a plurality of tubes 6 through a plurality of openings and tubular portions. For example, in a ventricular assist device therapy, two tubes 6 (cannulas) consisting of a transfusion tube and a blood drawing tube are placed in a patient. When the pad 5 has two openings 5a and the flanges 3 and 4 have two openings 3a and two openings 4a, respectively, and two tubular portions 3b, one cuff member or cuff member unit can hold two insert tubes. This may reduce aggression against a patient.

The first flange 3, the tubular portion 3b, and the second flange 4 are formed of a porous resin material that exhibits excellent adhesion to body tissue as described below. This porous resin has a three-dimensional network porous structure. The tubular portion 3b and the first flange 3 are combined into one piece.

The first flange 3 may have a planar shape of a circle, an ellipse, a lens, or a teardrop. In general, when skin is incised in a straight line with a scalpel, elliptical body tissue appears as illustrated in Fig, 1. Thus, the first flange 3 is preferably elliptical to cover the exposed tissue efficiently. The thickness of the first flange 3 depends on the physical strength of the first flange 3 as well as complicated factors, such as the average pore size and the inclination of the first flange 3 (these affect the depth of tissue invasion and the degree of differentiation) as described below. In general, the first flange 3 suitably has a thickness of about 0.2 to 50 mm, preferably about 0.2 to 10 mm, and more preferably about 1 to 7 mm. When the first flange 3 is circular, the first flange 3 suitably has a diameter of about 10 to 200 mm. When the first flange 3 is elliptical, lens-shaped, or teardrop-shaped, the major axis is preferably 10 to 200 mm and the minor axis is preferably about 5 to 80% of the major axis.

The tubular portion 3b suitably has a length of about 10 to 500 mm. The thickness of the tubular portion 3b depends on the physical strength of the tubular portion 3b as well as complicated factors, such as the average pore size and the inclination of the first flange 3 (these affect the depth of tissue invasion and the degree of differentiation) as described below. In general, the tubular portion 3b suitably has a thickness of about 0.05 to 20 mm. The tubular portion 3b is not necessarily straight and may be bent freely from an insertion site along a tube.

The second flange 4 is slightly smaller than the first flange 3. The outer edge of the first flange 3 extends beyond the outer edge of the second flange 4 preferably by 10 to 20 mm and more preferably by about 15 mm.

The second flange 4 has a concavity 4b. The pad 5 is fit into the concavity 4b.

Preferably, the second flange 4 extends beyond the outer edge of the pad 5 by about 0.1 to 30 mm. The amount by which the second flange 4 extends beyond the outer edge of the pad 5 may vary in the thickness direction on the second flange 4. For example, the amount may be 3 mm or less at the top surface of the pad and 1 mm to 30 mm at the bottom surface of the pad in contact with the first flange 3.

The material and the thickness of the pad 5 are the same as in the first aspect.

The flanges 3 and 4 and the pad 5 are stacked and are combined into one piece by an adhesive or the like.

The tube 6 is inserted through the openings 5a, 4a, and 3a and the tubular portion 3b. The tube 6 may be bonded in a watertight manner to the pad 5 by fusion using a highfrequency wave, heat, laser, and an ultrasonic wave or with an adhesive. The pad 5 and part of or the entire tube 6 may be integrally molded by injection molding. In this embodiment, the pad 5 and the tube 6 securely adhere to each other with an adhesive 7.

As illustrated in Fig. 11, when the tube 6 is inserted into a living body using the cuff member 2', skin is incised to expose body tissue. A small portion of subcutaneous tissue at the outer edge of the exposed body tissue is peeled off to form a pocket. The body tissue is incised to insert the tube 6 into the body tissue. Together with the tube 6, the tubular portion 3b is embedded in the body tissue. The incised body tissue around the tube 6 is sutured when necessary. The first flange 3 overlies the exposed body tissue and is embedded in the pocket formed by peeling off the subcutaneous tissue, that is, is placed under the epidermis. A second flange 4 is laid on the edge of skin around the exposed body tissue. The cuff member 2' may be fixed on the body surface of a patient by sewing the second flange 4 and epidermis together. Because the second flange is formed of a soft three-dimensional network structure material, it can easily be sutured with an ordinary suture needle. Furthermore, an adhesive tape that is air permeable and is imperviousness to water (not shown) may be attached over the outer edge of the second flange 4 and skin around this outer edge. The adhesive tape can prevent water or other liquids from entering the body tissue.

As described above, when the tube 6 is inserted into the body tissue using the cuff member 2', the downgrowth of skin initially tends to proceed to the interface between the second flange 4 and the first flange. However, because the interface has no physical space for epidermis to enter, the downgrowth of skin tends to proceed in the direction of the outer edge of the first flange and eventually under the first flange. In other words, the epidermis tends to grow apart from the sutured portion. However, in the cuff member 2' according to the present invention, tissue that invaded the first flange from the body tissue passes through the first flange and reaches the back of the epidermis. Thus, the epidermis linked to the body tissue is significantly inhibited from growing apart from the sutured portion on the first flange, and the epidermal end is maintained in close connection with the second flange.

While the tube 6 extends perpendicular to the outer surface of the body in Figs. 10a, 10b, and 11, it may obliquely extend as in a tube 6A illustrated in Fig. 12 or extend along the pad 5 as in a tube 6B illustrated in Figs. 13a and 13b. This may be properly determined by a person skilled in the art by consideration of the relationship between patient's posture and medical equipment to which an insert tube is connected and the weight and the width of movement of the tube 6, 6A, or 6B. In Fig. 13b, a pipe 5g for guiding the tube is combined with the pad 5.

A suitable material for the cuff member is described below.

A three-dimensional network open-cell porous structure composed of a thermoplastic resin or a thermosetting resin and constituting the flange 3, the first flange 3, and the tubular portion 3b of the cuff members according to the first aspect and the second aspect may have an average pore size of 50 to 1000 µm, particularly 100 to 1000 µm, and an apparent density of 0.01 to 0.5 g/cm³. The three-dimensional network open-cell porous structure may have similar structures over the entire surface of the transverse cross-section, or may have different structures on each side. The three-dimensional network open-cell porous structure may partly have a different average pore size and a different apparent density. For example, the average pore size or the apparent density may vary gradually from one side to the other side. In other words, the three-dimensional network open-cell porous structure may have anisotropy. When a cuff member has different average pore sizes in the thickness direction, preferably, the average pore size is larger on a surface in contact with body tissue and is smaller in a deep part. The reason is as follows: in general, a tissue invading a surface in contact with body tissue stably reaches a depth of about 10 mm in the thickness direction. However, a cell in a deep part may necrotize or be insufficient in differentiation even when a new blood vessel formed in the porous material has matured. Thus, tissue invasion is preferably inhibited by reducing the pore size in a part having a depth of more than about 10 mm.

The three-dimensional network open-cell porous structure may have large pores having a pore size much larger than the average pore size on a surface in contact with body tissue. Preferably, the large pores have a pore size of about 500 to 2000 µm. These pores existing near the surface layer on a body tissue side facilitate uniform impregnation with extracellular matrix such as collagen from the surface layer to a deep part. These pores also facilitate the invasion of cells from tissue and vascularization of capillary vessels. However, the pores having such a large diameter are not introduced into the concept of calculating the average pore size of the three-dimensional network porous structure according to the present invention.

The three-dimensional network porous structure of the first flange 3 and the tubular portion 3b has an average pore size of 50 to 1000 µm, particularly 100 to 1000 µm and an apparent density of 0.01 to 0.5 g/cm³. The average pore size is preferably 150 to 600 µm and more preferably 200 to 500 µm. The apparent density of 0.01 to 0.5 g/cm³ allows for satisfactory cell engraftment, an excellent physical strength, and elastic characteristics similar to those of subcutaneous tissue after invasion, engraftment, and organization of cells. The apparent density is preferably 0.03 to 0.3 g/cm³ and more preferably 0.05 to 0.2 g/cm³.

Even when the average pore size is constant, in the pore size distribution, the contribution ratio of pores having a pore size of 150 to 400 µm, which size is important for the cell invasion, is preferably high. Preferably, the contribution ratio of pores having a pore size of 150 to 400 µm is at least 10%, preferably at least 20%, more preferably at least 30%, still more preferably at least 40%, and still more preferably at least 50%. These contribution ratios enhance the cell invasion and the bonding and growth of the invading cells.

The contribution ratio of pores having a pore size of 150 to 400 µm to the average pore size in the three-dimensional network porous structure refers to the ratio of the number of pores having a pore size of 150 to 400 µm to the total number of pores, as determined by the measurement of the average pore size described in Example 1 below.

The three-dimensional network porous structure having the average pore size, the apparent density, and the pore size distribution as described above enhances the cell invasion in pores, bonding and growth of cells, and vascularization of capillary vessels. With this three-dimensional network porous structure, a cuff member can exhibit robust adhesion between subcutaneous tissue and a catheter or a cannula at an insertion point.

The second flange 4 is described below.

Preferably, the three-dimensional network porous structure of the second flange 4 has an average pore size of 5 to 80 µm and an apparent density of 0.1 to 0.7 g/cm³. More preferably, the three-dimensional network porous structure has an average pore size of 10 to 70 µm and an apparent density of 0.1 to 0.5 g/cm³.

Desirably, the three-dimensional network porous structure of the second flange 4 has a contribution ratio of pores having a pore size of 30 to 60 µm to the average pore size of at least 10%, particularly at least 30%, and more particularly at least 50%.

Desirably, the second flange 4 has a thickness of 0.1 to 10 mm and particularly 0.5 to 5 mm.

Examples of a thermoplastic resin or a thermosetting resin constituting the three-dimensional network porous structure include one or at least two of a polyurethane resin, a polyamide resin, a polylactic acid resin, a polyolefin resin, a polyester resin, a fluorocarbon resin, a urea resin, a phenol resin, an epoxy resin, a polyimide resin, an acrylic resin, a methacrylate resin, and their derivatives. A polyurethane resin is preferred. Among others, a segmented polyurethane resin is suitable.

A segmented polyurethane resin is synthesized from three components consisting of a polyol, a diisocyanate, and a chain-extending agent. The segmented polyurethane resin has elastomeric characteristics resulting from a block polymer structure containing a so-called hard segment and a so-called soft segment within the molecule. The elastic characteristics achieved by such a segmented polyurethane resin is expected to relief the stress generated at the interface between subcutaneous tissue and the cuff member when a patient, a catheter, or a cannula moves or when skin around an insertion point moves during disinfection or the like.

A cuff member according to the present invention may include a first layer including the three-dimensional network porous structure and a second layer disposed on the first layer. The second layer has a structure different from that of the first layer. The second layer may be a fiber assembly, a flexible film, or a three-dimensional network porous structure layer having an average pore size and an apparent density different from those of the three-dimensional network porous structure of the first layer.

Preferably, a nonwoven fabric or a woven fabric has a porosity of 100 to 5000 cc/cm²/min in view of flexibility, the suture strength with subcutaneous tissue, and the like. The porosity is determined according to JIS L 1004 and is sometimes called air permeability or air flow rate.

The fiber assembly may be composed of a synthetic resin of one or at least two selected from the group consisting of a polyurethane resin, a polyamide resin, a polylactic acid resin, a polyolefin resin, a polyester resin, a fluorocarbon resin, an acrylic resin, a methacrylate resin, and their derivatives. The fiber assembly may also be composed of naturally-occurring fiber of one or at least two selected from fibroin, chitin, chitosan, cellulose, and their derivatives. The fiber assembly may be composed of synthetic fiber and naturally-occurring fiber.

Examples of the flexible film include a thermoplastic resin film and specifically a film composed of one or at least two selected from the group consisting of a polyurethane resin, a polyamide resin, a polylactic acid resin, a polyolefin resin, a polyester resin, a fluorocarbon resin, a urea resin, a phenol resin, an epoxy resin, a polyimide resin, an acrylic resin, a methacrylate resin, and their derivatives. Preferably, examples of the flexible film include a film composed of one or at least two selected from the group consisting of a polyester resin, a fluorocarbon resin, a polyurethane resin, an acrylic resin, vinyl chloride, a fluorocarbon resin, and a silicon resin.

The flexible film has a thickness preferably of 0.1 to 500 µm, particularly of 0.1 to 100 µm, more particularly of 0.1 to 50 µm, and optimally of 0.1 to 10 µm.

The flexible film may be not only a solid film but also a porous film or a foam. A cuff member including a solid flexible film has an excellent antibacterial property and is therefore advantageous to control infection.

When a three-dimensional network porous structure having an average pore size and an apparent density different from those of the three-dimensional network porous structure of the first layer is used as the second layer, the three-dimensional network porous structure of the second layer may has an average pore size of 0.1 to 200 µm, an apparent density of 0.01 to 1.0 g/cm³, and a thickness of about 0.2 to 20 mm.

When the second layer is a fiber assembly, a flexible film, or a three-dimensional network porous structure layer having an average pore size and an apparent density different from those of the three-dimensional network porous structure of the first layer, examples of a method for stacking the second layer on a three-dimensional network porous structure layer include bonding using an adhesive and in particular thermocompression bonding of the first layer and the second layer with a hot-melt nonwoven fabric interposed therebetween. Examples of the hot-melt nonwoven fabric include a thermal adhesive polyamide sheet such as PA1001 from Nitto Boseki Co., Ltd. Other examples of a method for stacking the second layer on a three-dimensional network porous structure layer include a bonding method in which a surface layer of a contact surface is dissolved with a solvent, a bonding method in which a surface layer is melted by heat, and a method using an ultrasonic wave or a high frequency wave. Furthermore, the stacked layers may be continuously formed by stacking and molding a polymer dope and a fiber assembly or a flexible film following the production of the first layer.

The second layer may be composed of at least two sublayers selected from a fiber assembly, a flexible film, and a three-dimensional network porous structure layer. The three-dimensional network porous structure layer of the first layer may be stacked on the second layer to form a three-layer structure.

The three-dimensional network porous structures of the cuff members according to the first aspect and the second aspect may contain one or at least two selected from the group consisting of collagen type I, collagen type II, collagen type III, collagen type IV, atelocollagen, fibronectin, gelatin, hyaluronic acid, heparin, keratan acid, chondroitin, chondroitin sulfate, chondroitin sulfate B, elastin, heparan sulfate, laminin, thrombospondin, vitronectin, osteonectin, entactin, a copolymer of hydroxyethyl methacrylate and dimethylaminoethyl methacrylate, a copolymer of hydroxyethyl methacrylate and methacrylic acid, alginic acid, polyacrylamide, polydimethylacrylamide, and polyvinylpyrrolidone. The three-dimensional network porous structures of the cuff members according to the first aspect and the second aspect may further contain one or at least two selected from the group consisting of a platelet-derived growth factor, an epidermal growth factor, a transforming growth factor α, an insulin-like growth factor, an insulin-like growth factor binding protein, a hepatocyte growth factor, a vascular endothelial growth factor, angiopoietin, a nerve growth factor, a brain-derived neurotrophic factor, a ciliary neurotrophic factor, a transforming growth factor β, a latent transforming growth factor β, activin, a bone morphogenetic protein, a fibroblast growth factor, a tumor growth factor β, a diploid fibroblast growth factor, a heparin-binding epidermal growth factor-like growth factor, a schwannoma-derived growth factor, amphiregulin, betacellulin, epiregulin, lymphotoxin, erythropoietin, a tumor necrosis factor α, interleukin-1β, interleukin-6, interleukin-8, interleukin-17, interferon, an antiviral agent, an antimicrobial agent, and an antibiotic. In addition, the three-dimensional network porous structures of the cuff members according to the first aspect and the second aspect may be bonded with one type or at least two types of cells selected from the group consisting of an (optionally differentiated) embryonic stem cell, a vascular endothelial cell, a mesodermal cell, a smooth muscle cell, a peripheral blood vessel cell, and a mesothelial cell.

In the cuff member according to the first aspect and the second aspect, a thermoplastic resin skeleton or a thermosetting resin skeleton constituting the three-dimensional network porous structure layer may also have micropores. Such micropores provide a skeleton surface having complicated bumps and dips rather than a smooth surface and are thereby effective in holding collagen, a cell growth factor, or the like. This can result in an increase in cell engraftment. However, the micropores are not introduced into the concept of calculating the average pore size of the three-dimensional network porous structure layer according to the present invention.

An example of a method for manufacturing a three-dimensional network porous structure composed of a thermoplastic polyurethane resin and constituting a cuff member according to the present invention will be described below. A method for manufacturing a cuff member according to the present invention is not limited to the following method.

To manufacture a three-dimensional network porous structure formed of a thermoplastic polyurethane resin, first, a polyurethane resin, a water-soluble polymer compound serving as a pore-forming agent as described below, and an organic solvent that is a good solvent for the polyurethane resin are mixed to produce a polymer dope. More specifically, after the polyurethane resin is mixed with the organic solvent to yield a homogeneous solution, the water-soluble polymer compound is dispersed in the solution. Examples of the organic solvent include N,N-dimethylformamide, N-methyl-2-pyrrolidinone, and tetrahydrofuran. The organic solvent is not limited thereto and may be any organic solvent that can dissolve a thermoplastic polyurethane resin. Alternatively, the polyurethane resin may be melted by heat in the presence of a reduced amount of organic solvent or in the absence of an organic solvent and then is mixed with the pore-forming agent.

Examples of the water-soluble polymer compound serving as a pore-forming agent include polyethylene glycol, polypropylene glycol, polyvinyl alcohol, polyvinylpyrrolidone, alginic acid, carboxymethyl cellulose, hydroxypropyl cellulose, methyl cellulose, and ethyl cellulose. The water-soluble polymer compound is not limited thereto and may be any water-soluble polymer compound that can homogeneously be dispersed together with a thermoplastic resin to form a polymer dope. In addition, depending on the type of thermoplastic resin, a lipophilic compound, such as a phthalate ester or paraffin, and an inorganic salt, such as lithium chloride and calcium carbonate, may be used instead of the water-soluble polymer compound. Furthermore, a nucleating agent for a polymer may also be utilized to enhance the generation of secondary particles, that is, the formation of a skeleton of the porous material during solidification.

The polymer dope prepared from the thermoplastic polyurethane resin, the organic solvent, and the water-soluble polymer compound is then dipped in a solidification bath containing a poor solvent for the thermoplastic polyurethane resin to extract and remove the organic solvent and the water-soluble polymer compound in the solidification bath. Removing part or all of the organic solvent and the water-soluble polymer compound can yield a three-dimensional network porous structure material formed of the polyurethane resin. Examples of the poor solvent used herein include water, a lower alcohol, and a low carbon number ketone. The solidified polyurethane resin may finally be washed with water or the like to remove the remaining organic solvent and the pore-forming agent.

### EXAMPLES AND COMPARATIVE EXAMPLES

While the present invention will be more specifically described below with reference to examples and a comparative example, the present invention is not limited to the following examples within the gist of the present invention.

In Example 1, a cuff member having a shape illustrated in Fig. 1b according to the first aspect was manufactured to perform an implantation experiment in a goat. An animal experiment was appropriately performed according to an international standard with attention to an ethical aspect.

### [Example 1]

### <Manufacture of porous material>

A thermoplastic polyurethane resin (Nippon Miractran Co., Ltd., Miractran E980PNAT) was dissolved in N-methyl-2-pyrrolidinone (Kanto Chemical Co., Inc., reagent for peptide synthesis, NMP) at room temperature with a dissolver (about 2,000 rpm) to yield 12.5% solution (weight/weight). About 1.0 kg of the NMP solution was charged into a planetary mill (Inoue Manufacturing Co., Ltd., capacity of 2.0 L, PLM-2). Methylcellulose (Kanto Chemical Co., Inc., reagent, 50 cp grade) the amount of which corresponds to half the weight of the polyurethane resin was added to the NMP solution and was stirred at 60°C for 120 min. The solution was continuously stirred and was degassed under vacuum at 20 mmHg (2.7 kPa) for 10 min to yield a polymer dope.

Two 150 mm x 150 mm square Teflon frames having a thickness of 3 mm and an opening of 140 mm x 140 mm were stacked and were fixed with a square filter paper for a chemical experiment (Toyo Roshi Kaisha, Ltd., for quantitative analysis, No. 2) having a size of 150 mm x 150 mm interposed therebetween. The polymer dope was poured into the frame unit. An excessive amount of polymer dope was removed with a glass rod. Then, a 150 mm x 150 mm square filter paper for a chemical experiment (Toyo Roshi Kaisha, Ltd., for quantitative analysis, No. 2) was placed on the frame unit and was fixed. The frame unit was immersed in methanol under reflux for 72 hours to extract and remove NMP from the filter papers for a chemical experiment disposed on both sides of the frame unit and thereby solidify the polyurethane resin. Methanol was continuously refluxed and was replaced with a fresh methanol every 20 min.

After 72 hours, a solidified polyurethane resin was removed from the fluorocarbon resin frame and was washed in Japanese Pharmacopoeia purified water for 72 hours to extract and remove methylcellulose, methanol, and the remaining NMP. The product was dried at room temperature under vacuum (20 mmHg) for 24 hours to yield a three-dimensional network porous structure material formed of the thermoplastic polyurethane resin.

The average pore size and the apparent density of the resulting three-dimensional network porous structure material were measured according to the following methods. A specimen was cut at room temperature with a double-edged blade (Feather Safety Razor Co., Ltd., High Stainless).

### [Measurement of average pore size]

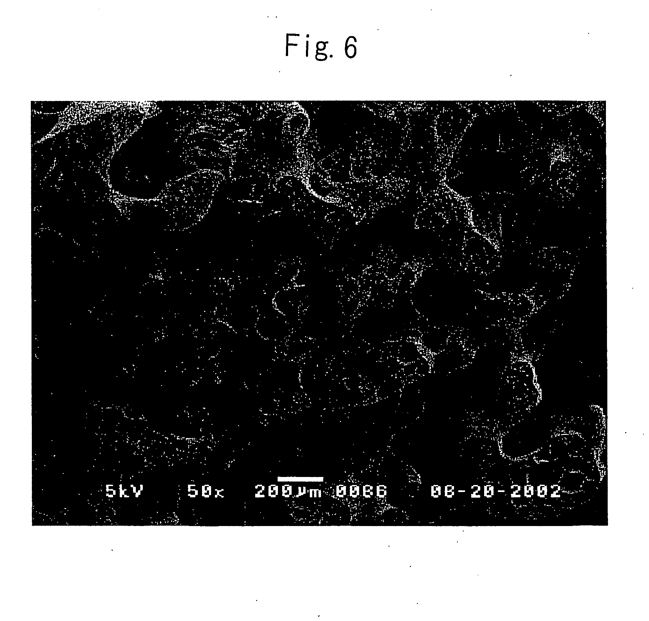

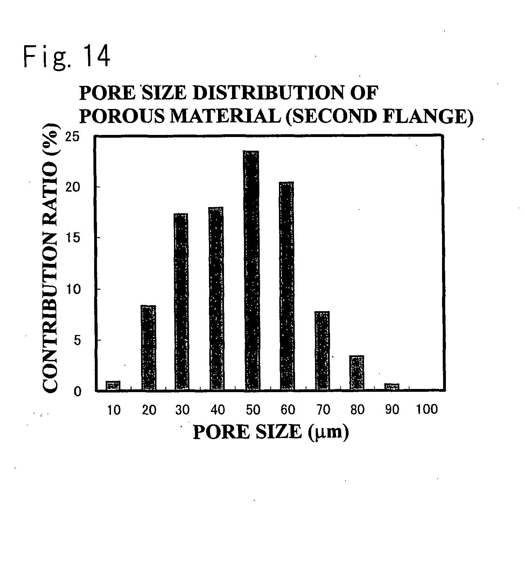

A photograph of a plane (section) of the specimen cut with the double-edged blade was taken with an electron microscope (Topcon Corporation, SM200) (representative example was illustrated in Fig. 6) and was subjected to image processing (LUZEX AP from Nireco Corporation was used as an image analyzing system, and LE N50 from Sony Corporation was used as a CCD camera for image capture). Pores in the same plane were defined as individual figures surrounded by a three-dimensional network structure skeleton. The areas of the individual figures were measured. The areas were converted into perfect circle areas. The diameter of the corresponding circle was determined as the pore size. Micropores in the porous material skeleton formed by phase separation during the formation of the porous material were discounted and only communicating holes in the same plane were measured. At the same time, the pore size distribution of all the measured pores was determined as illustrated in Fig. 14. The contribution ratio of pores having a pore size of 150 to 400 µm was determined from the pore size distribution. The average pore size of the porous structure was 286.1 µm. The contribution ratio of pores having a pore size of 150 to 400 µm was 87.6%.

### [Measurement of apparent density]

A porous structure was cut into an about 10 mm x 10 mm x 3 mm rectangular parallelepiped with a double-edged blade. The volume of the rectangular parallelepiped was determined from dimensions measured with a projector (Nikon, V-12).
The apparent density of the rectangular parallelepiped was determined by dividing the weight by the volume and was 0.118 ± 0.006 g/cm³.

### <Molding of cuff member>

The porous structure was cut into a flange 3 illustrated in Fig. 1 with a Thompson punching blade (the longer diameter was 120 mm and the shorter diameter was 70 mm). Then, by the same method as described above, the thermoplastic polyurethane resin was molded into a tubular three-dimensional network porous structure material (inner diameter 7.7 mm, outer diameter 13.5 mm, and length 50 mm) (tubular portion 3b in Fig. 1). Subsequently, the thermoplastic polyurethane resin was heat-pressed by a routine method into a mirror-finished sheet having a thickness of 2.0 mm, which was cut into a pad 5 (the longer diameter was 100 mm and the shorter diameter was 50 mm) with a Thompson punching blade.

Tetrahydrofuran (Kanto Chemical Co., Inc., reagent, guaranteed reagent) was applied to the surface of the pad. The flange formed of the three-dimensional network porous structure material was placed on the pad and was pressed at a load of 1.0 kg/cm². Openings 5a and 3a were bored at the center of the pad and the flange.

A tube 6 (inner diameter 5.0 mm and outer diameter 7.7 mm) was inserted into the openings and was fixed. A tubular portion 3b was securely attached to the undersurface of the flange (reference numeral 3 in Fig. 1) to surround the tube 6. A cuff member according to the first aspect was thus manufactured.

Then, the cuff member was subcutaneously embedded in a goat.
Two adult goats (both were female and the body weights were 54 kg and 53 kg) were used as test subjects. The test region extended from a shaved left chest to abdominal epidermis. During a surgical operation, an endotracheal tube was immediately inserted into a subject in the left lateral decubitus position according to common manipulation. The subject was maintained under general anesthesia with isoflurane. After epidermis around the chest and the abdomen was disinfected with Isodine, epidermis was incised about 100 mm. A cuff member according to the present invention sterilized with an ethylene oxide gas was placed under the epidermis. The circumference of a cuff member 2 extending beyond the pad 5 by about 10 mm was placed under the epidermis. The cuff member was fixed by suturing subcutaneous tissue while the circumference of the pad 5 and the end of the incised epidermis were put together.

After operation, the test region was disinfected with an acid water twice a day. The subject took water freely and was fed with a proper amount (about 1 kg) of hay cubes five times a day. After one and two postoperative months, the embedded test specimen and its surrounding tissue were removed under general anesthesia.

The test specimen was intimately engrafted on the surrounding tissue. They were difficult to separate from each other. There were no signs of infection, inflammation, or the like around the test specimen.

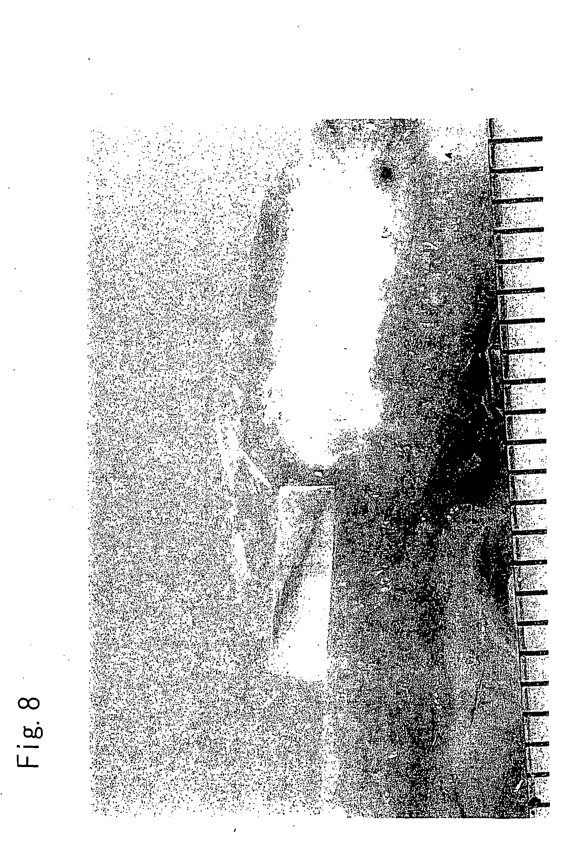

Fig. 8 is a cross-sectional photograph taken through a magnifier in which a boundary line between the pad 5 and the epidermis of an adult goat on the embedded flange 3 was observed. The edge of the pad 5 and the epidermis were stably bonded together while only a very small gap was present therebetween. As described above with reference to Fig. 2, since the flange 3 of the cuff member adhered to subcutaneous tissue, the epidermis did not even reach the contact portion between the tube 6 and subcutaneous tissue. The downgrowth phenomenon was completely inhibited.

The removed test specimen was immediately fixed with 10% neutral buffered formalin. An HE stained specimen was prepared by an ordinary method and was observed under an optical microscope. As histological findings, a three-dimensional network porous structure layer constituting the flange 3 and the tubular portion 3b of a cuff member unit according to the present invention was invaded by extracellular matrix-based granulation tissue, such as fibroblast, macrophage, or collagen fiber, extending from the surrounding tissue. Many new blood vessels were observed in the three-dimensional network porous structure layer. Autopsy after one month and two months showed that the invaded tissues changed into mature connective tissues over time.

This example indicated that the cuff member according to the first aspect was organized through the invasion of the flange 3 and the tubular portion 3b by biological cells, isolated a wound from the outside, and blocked exacerbating factors such as bacterial infection during healing.

### [Comparative Example 1]

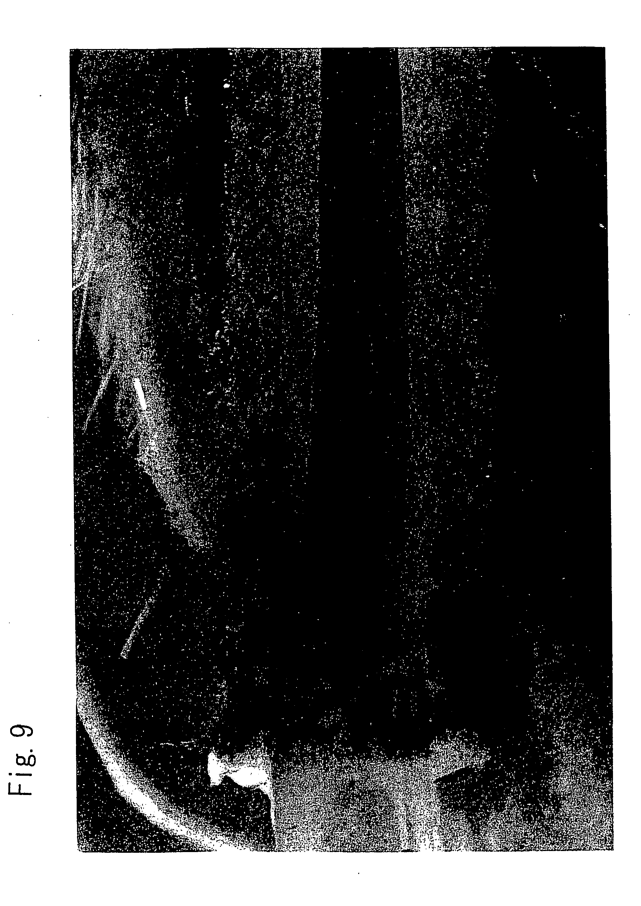

A cuff member was manufactured by removing the pad 5 and the flange 3 from the cuff member unit according to the first aspect and only attaching a tubular portion 3b to a tube 6 (inner diameter 2.0 mm and outer diameter 3.0 mm). This cuff member was implanted in an adult goat by the same method as in Example 1 while the upper end of the tubular portion 3b was exposed about 2 mm from epidermis. After one month, the test specimen and its surrounding tissue were removed as in Example 1. In a deep part of subcutaneous tissue, the test specimen was intimately engrafted on its surrounding tissue, and they were difficult to separate from each other. However, in a part near epidermis, the test specimen was only slightly engrafted on the surrounding tissue, and they were easily separated from each other. Fig. 9 is a cross sectional photograph of the test specimen and the surrounding tissue, taken through a magnifier. In a part near epidermis, signs of infection, inflammation, or the like were observed. Furthermore, as illustrated in Fig. 3, the downgrowth phenomenon was observed along the tubular portion 3b. This is in good agreement with the peeling characteristic of the test specimen and tissue.
Disinfection was necessary twice a day to remove sebum or the like accumulated at an insertion point. Without this careful treatment, infection occurred. Furthermore, the cuff member was easily pulled out by hand.

### [Example 2]

### Example 2 according to the second aspect will be described below.

### <Manufacture of porous material for first flange and tubular portion>

### A polymer dope was prepared as in Example 1.

Two 150 mm x 150 mm square fluorocarbon resin frames having a thickness of 5 mm and an opening of 140 mm x 140 mm were stacked and were fixed with a square filter paper for a chemical experiment (Toyo Roshi Kaisha, Ltd., for quantitative analysis, No. 2) having a size of 150 mm x 150 mm interposed therebetween. The polymer dope was poured into the frame. An excessive amount of polymer dope was removed with a glass rod. Then, a 150 mm x 150 mm square filter paper for a chemical experiment (Toyo Roshi Kaisha, Ltd., for quantitative analysis, No. 2) was placed on the frame unit and was fixed. The frame unit was immersed in methanol under reflux for 72 hours to extract and remove NMP from the filter papers for a chemical experiment disposed on both sides of the frame unit and thereby solidify the polyurethane resin. Methanol was continuously refluxed and was replaced with a fresh methanol every 20 min.

After 72 hours, a solidified polyurethane resin was removed from the fluorocarbon resin frame and was washed in Japanese Pharmacopoeia purified water for 72 hours to extract and remove methylcellulose, methanol, and the remaining NMP. The product was dried at room temperature under vacuum (20 mmHg) for 24 hours to yield a three-dimensional network porous structure material formed of the thermoplastic polyurethane resin.

The average pore size and the apparent density of the resulting three-dimensional network porous structure material were measured according to the same methods as in Example 1 and were found to be the same as the porous material in Example 1.

### <Manufacture of porous material for second flange>

A porous structure of a second flange was molded as in the porous material of the first flange except that the NMP solution of Miractran E980PNAT was changed from 12.5% to 20.0% and the thickness of the square fluorocarbon resin frame was changed to 4 mm. The average pore size and the apparent density were measured by the same methods as in the porous structure of the first flange. The average pore size was 41.7 µm. The pore size distribution was illustrated in Fig. 14. The contribution ratio of pores having a pore size of 30 µm to 60 µm was 79.0%. The apparent density was 0.228 ± 0.011 g/cm³.

### <Molding of cuff member>

Miractran E980PNAT was molded into a mirror-finished solid sheet having a thickness of 2 mm with a heat press machine by a routine method. The sheet was cut into a perfect ellipse having a major axis of 40 mm and a minor axis of 30 mm with a Thompson blade to prepare a polymer resin pad 5.

Then, the porous structure of the second flange having a thickness of 4 mm was cut into a perfect ellipse having a major axis of 50 mm and a minor axis of 40 mm with a Thompson blade and was figured into a uniform and smooth porous sheet having a thickness of 3 mm. A perfect elliptical recess having a major axis of 40 mm, a minor axis of 30 mm, and a depth of 2 mm was formed at the center of the porous sheet. This product was processed into a second flange 4. In this process, cutting was performed on the basis of a CAD drawing with an NC machine (Roland DG Corporation, PNC-3200).

After tetrahydrofuran (Kanto Chemical Co., Inc., reagent, guaranteed reagent) was applied to the polymer resin pad, the polymer resin pad was placed in the central recess of the second flange and was press-bonded at a load of 1.0 kg/cm².

A porous structure of the first flange 3 was cut into a perfect ellipse having a major axis of 70 mm and a minor axis of 60 mm with a Thompson blade. Subsequently, after tetrahydrofuran is applied to a side of the second flange 4 to which no polymer resin pad was bonded, the second flange 4 was placed on the first flange and was pressed at a load of 1.0 kg/cm². Central openings 5a, 4a, and 3a were bored. A tube 6 (inner diameter 5.0 mm and outer diameter 7.7 mm) was inserted into the openings and was fixed.

Then, by the same method as described above, a thermoplastic polyurethane resin was molded into a tubular three-dimensional network porous structure material (inner diameter 7.7 mm, outer diameter 13.5 mm, and length 50 mm) to form a tubular portion 3b. The tubular portion 3b was securely attached to the undersurface of the first flange 3 to surround the tube 6. A cuff member according to the second aspect was thus manufactured.

Then, the cuff member was subcutaneously embedded in a goat.

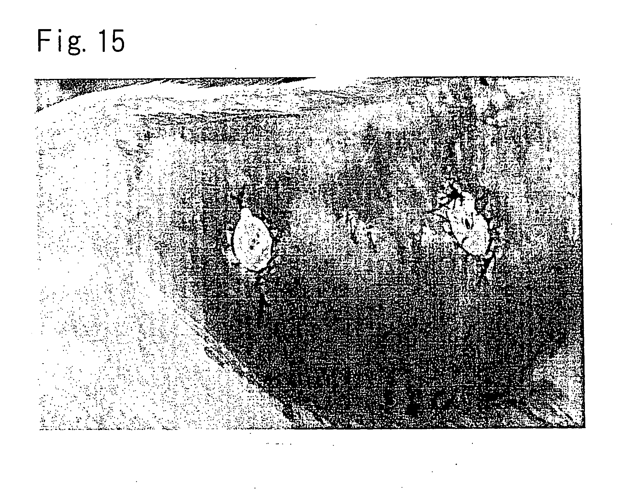

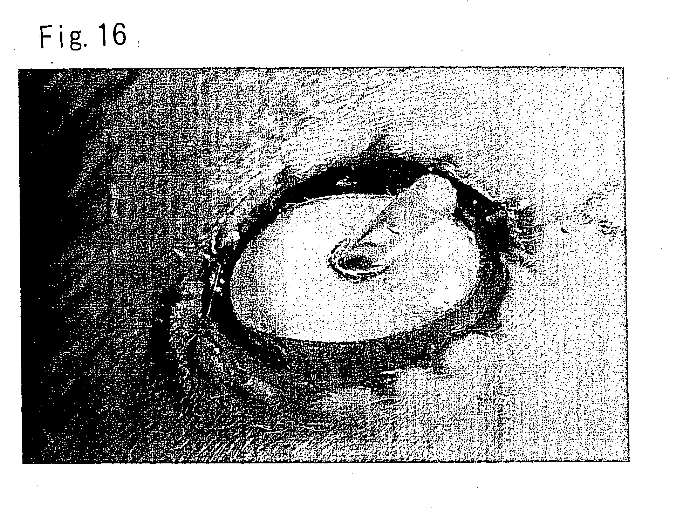

Two adult goats (both were female and the body weights were 54 kg and 53 kg) were used as test subjects. The test region extended from a shaved left chest to abdominal epidermis. During a surgical operation, an endotracheal tube was immediately inserted into the subject in the left lateral decubitus position according to common manipulation. The subject was maintained under general anesthesia with isoflurane. After epidermis around the chest and the abdomen was disinfected with Isodine, the epidermis was incised about 100 mm. A cuff member according to the present invention manufactured in Example 1 was sterilized with an ethylene oxide gas and was placed under the epidermis (so that the circumference of the first flange 3 extending beyond the pad 5 and the second flange 4 was placed under the epidermis). The cuff member was fixed by suturing subcutaneous tissue while the circumference of the second flange 4 and the end of the incised epidermis were put together (Fig. 15). For the first postoperative week, the sutured portion was disinfected with an acid water twice a day. The subject took water freely and was fed with a proper amount (about 1 kg) of hay cubes five times a day. After the first postoperative week, while no disinfection was performed and no antibiotic was administered, the subject was progressing favorably without any sign of infection or the like. Fig. 16 is a photograph of the interface between the second flange and epidermis of an adult goat.

Fig. 16 showed that the edge of the second flange 4 and the epidermis were stably bonded together while only a very small gap was present therebetween. Since the first flange 3 adhered to subcutaneous tissue, the epidermis did not even reach the contact portion of the tube 6 and subcutaneous tissue. Thus, the downgrowth phenomenon was completely inhibited.

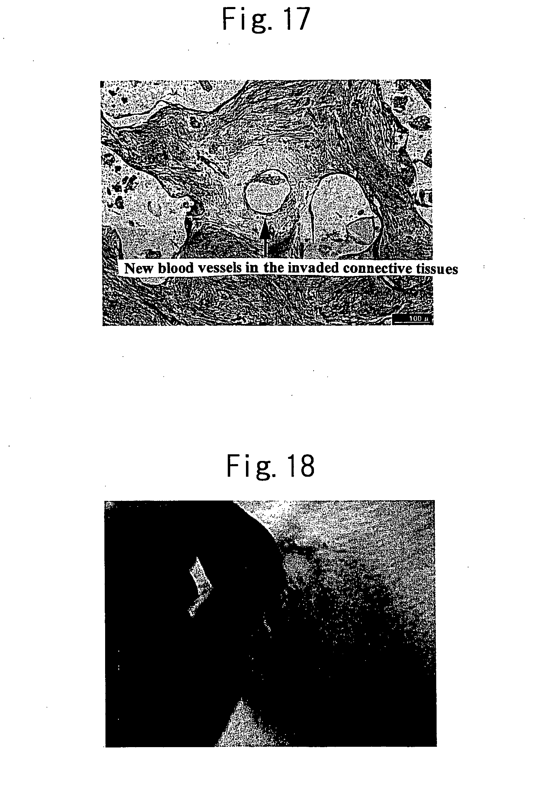

Then, after one, three, six, and 12 postoperative months, the embedded test specimen and the surrounding tissue were removed under general anesthesia. Even after the first postoperative month, the test specimen was intimately engrafted on the surrounding tissue, and they were difficult to separate from each other. The adhesion became robust with time. After the removal, macroscopic pathological findings, such as infection and inflammation, were not observed around the implanted cuff member. The removed test specimen was immediately fixed with 10% neutral buffered formalin. An HE stained specimen was prepared by an ordinary method and was observed under an optical microscope. As histological findings, a three-dimensional network porous structure layer constituting the first flange 3 and the tubular portion 3b of the cuff member unit according to the present invention was invaded by extracellular matrix-based granulation tissue, such as fibroblast, macrophage, or collagen fiber, extending from the surrounding tissue. Many new blood vessels were observed in the three-dimensional network porous structure layer (Fig. 17). Autopsy after one, three, and six months showed that the invaded tissues changed into mature connective tissues over time.

After three postoperative months, the adhesion of the test specimen to body tissue became more robust. Even when the cuff member was pulled by hand, the cuff member was not pulled out by hand, and subcutaneous tissue combined with the cuff member was raised (Fig. 18). During the test period, while no antibiotic was administered and no disinfection was performed, except that excess body hair was shaved, the subject supplied with water and feed was progressing favorably.

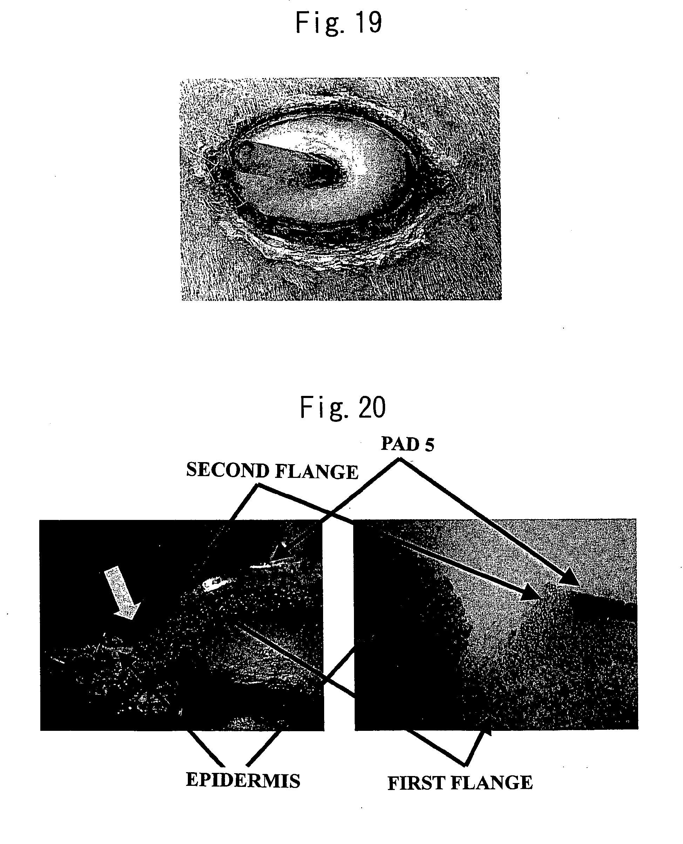

Even after 12 postoperative months, epidermis did not go back on the first flange and intimately adhered to the edge of the second flange. While no disinfection was performed for these 12 months, no infection was induced (Fig. 19).

A left photograph of Fig. 20 is a cross section of a cuff member specimen removed after 12 postoperative months. A right photograph of Fig. 20 is a tissue specimen in the same visual field. A tissue passing through the first flange reached the second flange. The tissue was linked and adhered to epidermis on the first flange. The epidermis intimately adhered to the edge of the second flange. No infection layer was observed at the interface between the epidermis and the second flange.

This example indicated that the cuff member unit according to the second aspect was organized through the invasion of the first flange 3 and the tubular portion 3b by biological cells, isolated a wound from the outside through the adhesion of the second flange to epidermis, and blocked exacerbating factors such as bacterial infection during healing.

## Claims

1. A cuff member comprising a three-dimensional network open-cell porous structure, comprising:
a flange overlying the outer surface of a living body; and
a tubular portion standing on one side of the flange,
wherein the three-dimensional network open-cell porous structure is formed of a base resin composed of a thermoplastic resin or a thermosetting resin and has an average pore size of 50 to 1000 µm and an apparent density of 0.01 to 0.5 g/cm³.

2. The cuff member according to Claim 1, wherein the three-dimensional network porous structure has an average pore size of 150 to 600 µm and an apparent density of 0.01 to 0.5 g/cm³.

3. The cuff member according to Claim 2, wherein the three-dimensional network porous structure has an average pore size of 200 to 500 µm and an apparent density of 0.01 to 0.5 g/cm³.

4. The cuff member according to Claim 1, wherein the three-dimensional network porous structure has an apparent density of 0.05 to 0.3 g/cm³.

5. The cuff member according to Claim 4, wherein the three-dimensional network porous structure has an apparent density of 0.05 to 0.2 g/cm³.

6. The cuff member according to Claim 1, wherein the contribution ratio of pores having a pore size of 150 to 400 µm to the average pore size in the three-dimensional network porous structure is at least 10%.

7. The cuff member according to Claim 1, wherein the contribution ratio of pores having a pore size of 150 to 400 µm to the average pore size in the three-dimensional network porous structure is at least 20%.

8. The cuff member according to Claim 1, wherein the contribution ratio of pores having a pore size of 150 to 400 µm to the average pore size in the three-dimensional network porous structure is at least 30%.

9. The cuff member according to Claim 1, wherein the contribution ratio of pores having a pore size of 150 to 400 µm to the average pore size in the three-dimensional network porous structure is at least 40%.

10. The cuff member according to Claim 1, wherein the contribution ratio of pores having a pore size of 150 to 400 µm to the average pore size in the three-dimensional network porous structure is at least 50%.

11. The cuff member according to Claim 1, wherein the three-dimensional network porous structure has a thickness of 0.2 to 500 mm.

12. The cuff member according to Claim 1, wherein the three-dimensional network porous structure has a thickness of 0.2 to 100 mm.

13. The cuff member according to Claim 1, wherein the three-dimensional network porous structure has a thickness of 0.2 to 50 mm.

14. The cuff member according to Claim 1, wherein the three-dimensional network porous structure has a thickness of 0.2 to 10 mm.

15. The cuff member according to Claim 1, wherein the three-dimensional network porous structure has a thickness of 0.2 to 5 mm.

16. The cuff member according to Claim 1, wherein the base resin is one or at least two selected from the group consisting of a polyurethane resin, a polyamide resin, a polylactic acid resin, a polyolefin resin, a polyester resin, a fluorocarbon resin, a urea resin, a phenol resin, an epoxy resin, a polyimide resin, an acrylic resin, a methacrylate resin, and their derivatives.

17. The cuff member according to Claim 16, wherein the base resin is a polyurethane resin.

18. The cuff member according to Claim 17, wherein the polyurethane resin is a segmented polyurethane resin.

19. The cuff member according to Claim 1, wherein the cuff member is a laminate of a first layer formed of the three-dimensional network porous structure and a second layer different from the first layer.

20. The cuff member according to Claim 19, wherein the second layer is one or at least two selected from the group consisting of a fiber assembly, a flexible film, and a three-dimensional network porous structure layer having an average pore size and/or an apparent density that is different from those of the three-dimensional network porous structure of the first layer.

21. The cuff member according to Claim 20, wherein the fiber assembly is a nonwoven fabric or a woven fabric.

22. The cuff member according to Claim 21, wherein the nonwoven fabric or the woven fabric has a thickness of 0.1 to 100 mm.

23. The cuff member according to Claim 22, wherein the nonwoven fabric or the woven fabric has a thickness of 0.1 to 50 mm.

24. The cuff member according to Claim 23, wherein the nonwoven fabric or the woven fabric has a thickness of 0.1 to 10 mm.

25. The cuff member according to Claim 24, wherein the nonwoven fabric or the woven fabric has a thickness of 0.1 to 5 mm.

26. The cuff member according to Claim 21, wherein the nonwoven fabric or the woven fabric has a porosity of 100 to 5000 cc/cm²/min.

27. The cuff member according to Claim 20, wherein the fiber assembly is composed of one or at least two selected from the group consisting of a polyurethane resin, a polyamide resin, a polylactic acid resin, a polyolefin resin, a polyester resin, a fluorocarbon resin, an acrylic resin, a methacrylate resin, and their derivatives.

28. The cuff member according to Claim 20, wherein the fiber assembly is composed of one or at least two selected from the group consisting of fibroin, chitin, chitosan, cellulose, and their derivatives.

29. The cuff member according to Claim 20, wherein the flexible film is a thermoplastic resin film.

30. The cuff member according to Claim 29, wherein the thermoplastic resin is one or at least two selected from the group consisting of a polyurethane resin, a polyamide resin, a polylactic acid resin, a polyolefin resin, a polyester resin, a fluorocarbon resin, a urea resin, a phenol resin, an epoxy resin, a polyimide resin, a silicon resin, an acrylic resin, a methacrylate resin, and their derivatives.

31. The cuff member according to Claim 29, wherein the thermoplastic resin is one or at least two selected from the group consisting of polyvinyl chloride, a polyurethane resin, a fluorocarbon resin, and a silicon resin.

32. The cuff member according to Claim 20, wherein the flexible film has a thickness of 0.1 to 500 µm.

33. The cuff member according to Claim 20, wherein the flexible film has a thickness of 0.1 to 100 µm.

34. The cuff member according to Claim 20, wherein the flexible film has a thickness of 0.1 to 50 µm.

35. The cuff member according to Claim 20, wherein the flexible film has a thickness of 0.1 to 10 µm.

36. The cuff member according to Claim 20, wherein the three-dimensional network porous structure layer of the second layer has an average pore size of 0.1 to 200 µm and an apparent density of 0.01 to 1.0 g/cm³.

37. The cuff member according to Claim 20, wherein the three-dimensional network porous structure layer of the second layer has a thickness of 0.2 to 20 mm.

38. The cuff member according to Claim 1, wherein the three-dimensional network porous structure comprises one or at least two selected from the group consisting of collagen type I, collagen type II, collagen type III, collagen type IV, atelocollagen, fibronectin, gelatin, hyaluronic acid, heparin, keratan acid, chondroitin, chondroitin sulfate, chondroitin sulfate B, elastin, heparan sulfate, laminin, thrombospondin, vitronectin, osteonectin, entactin, a copolymer of hydroxyethyl methacrylate and dimethylaminoethyl methacrylate, a copolymer of hydroxyethyl methacrylate and methacrylic acid, alginic acid, polyacrylamide, polydimethylacrylamide, and polyvinylpyrrolidone.

39. The cuff member according to Claim 38, wherein the three-dimensional network porous structure further comprises one or at least two selected from the group consisting of a platelet-derived growth factor, an epidermal growth factor, a transforming growth factor α, an insulin-like growth factor, an insulin-like growth factor binding protein, a hepatocyte growth factor, a vascular endothelial growth factor, angiopoietin, a nerve growth factor, a brain-derived neurotrophic factor, a ciliary neurotrophic factor, a transforming growth factor β, a latent transforming growth factor β, activin, a bone morphogenetic protein, a fibroblast growth factor, a tumor growth factor β, a diploid fibroblast growth factor, a heparin-binding epidermal growth factor-like growth factor, a schwannoma-derived growth factor, amphiregulin, betacellulin, epiregulin, lymphotoxin, erythropoietin, a tumor necrosis factor α, interleukin-1β, interleukin-6, interleukin-8, interleukin-17, interferon, an antiviral agent, an antimicrobial agent, and an antibiotic.

40. The cuff member according to Claim 39, wherein a cell is bonded to the three-dimensional network porous structure.

41. The cuff member according to Claim 40, wherein the cell is one or at least two selected from the group consisting of an embryonic stem cell, a vascular endothelial cell, a mesodermal cell, a smooth muscle cell, a peripheral blood vessel cell, and a mesothelial cell.

42. The cuff member according to Claim 41, wherein the embryonic stem cell is differentiated.

43. The cuff member according to Claim 1, further comprising:
a polymeric material pad overlying the other side of the flange of the cuff member.

44. The cuff member according to Claim 43, wherein a tube for supplying a fluid to a living body or draining a fluid from a living body passes through the pad, the flange, and the tubular portion of the cuff member.

45. The cuff member according to Claim 44, wherein the interface between the tube and the pad is hermetically sealed.

46. The cuff member according to Claim 1, wherein the flange is a first flange,
the cuff member further comprises a second flange one side of which overlies the other side of the first flange and a polymeric material pad overlying the other side of the second flange,
the first flange and the tubular portion comprise a three-dimensional network open-cell porous structure formed of a base resin composed of a thermoplastic resin or a thermosetting resin, the three-dimensional network open-cell porous structure having an average pore size of 100 to 1000 µm and an apparent density of 0.01 to 0.5 g/cm³, and
the second flange comprises a three-dimensional network open-cell porous structure formed of a base resin composed of a thermoplastic resin or a thermosetting resin, the three-dimensional network open-cell porous structure having an average pore size of 1 to 100 µm and an apparent density of 0.05 to 1 g/cm³.

47. The cuff member according to Claim 46, wherein the three-dimensional network porous structures of the first flange and the tubular portion have an average pore size of 150 to 600 µm and an apparent density of 0.03 to 0.3 g/cm³.

48. The cuff member according to Claim 46, wherein the three-dimensional network porous structures of the first flange and the tubular portion have an average pore size of 200 to 500 µm and an apparent density of 0.05 to 0.2 g/cm³.

49. The cuff member according to Claim 46, wherein the contribution ratio of pores having a pore size of 150 to 400 µm to the average pore size in the three-dimensional network porous structures of the first flange and the tubular portion is at least 10%.

50. The cuff member according to Claim 46, wherein the contribution ratio of pores having a pore size of 150 to 400 µm to the average pore size in the three-dimensional network porous structures of the first flange and the tubular portion is at least 30%.

51. The cuff member according to Claim 46, wherein the contribution ratio of pores having a pore size of 150 to 400 µm to the average pore size in the three-dimensional network porous structures of the first flange and the tubular portion is at least 50%.

52. The cuff member according to Claim 46, wherein the three-dimensional network porous structure of the first flange has a thickness of 0.2 to 50 mm.

53. The cuff member according to Claim 46, wherein the three-dimensional network porous structure of the first flange has a thickness of 0.2 to 10 mm.

54. The cuff member according to Claim 46, wherein the three-dimensional network porous structure of the first flange has a thickness of 1 to 7 mm.

55. The cuff member according to Claim 46, wherein the three-dimensional network porous structure of the second flange has an average pore size of 5 to 80 µm and an apparent density of 0.1 to 0.7 g/cm³.

56. The cuff member according to Claim 46, wherein the three-dimensional network porous structure of the second flange has an average pore size of 10 to 70 µm and an apparent density of 0.1 to 0.5 g/cm³.

57. The cuff member according to Claim 46, wherein the contribution ratio of pores having a pore size of 30 to 60 µm to the average pore size in the three-dimensional network porous structure of the second flange is at least 10%.

58. The cuff member according to Claim 46, wherein the contribution ratio of pores having a pore size of 30 to 60 µm to the average pore size in the three-dimensional network porous structure of the second flange is at least 30%.

59. The cuff member according to Claim 46, wherein the contribution ratio of pores having a pore size of 30 to 60 µm to the average pore size in the three-dimensional network porous structure of the second flange is at least 50%.

60. The cuff member according to Claim 46, wherein the three-dimensional network porous structure of the second flange has a thickness of 0.1 to 10.0 mm.

61. The cuff member according to Claim 46, wherein the three-dimensional network porous structure of the second flange has a thickness of 0.5 to 5 mm.

62. The cuff member according to Claim 46, wherein the second flange extends beyond the outer edge of the polymer resin pad and the first flange extends beyond the outer edge of the second flange.

63. The cuff member according to Claim 62, wherein the second flange extends beyond the outer edge of the polymer resin pad by 0.1 to 30 mm.

64. The cuff member according to Claim 62, wherein the second flange extends beyond the outer edge of the polymer resin pad by different amounts in the thickness direction of the second flange.

65. The cuff member according to Claim 62, wherein the second flange extends beyond the outer edge of the polymer resin pad by 3 mm or less at the top surface of the polymer resin pad and beyond the outer edge of the polymer resin pad by 1 mm to 30 mm at the bottom surface of the polymer resin pad in contact with the first flange.
